Europäisches Patentamt

European Patent Office

Office européen des brevets

(11) Veröffentlichungsnummer: **0 003 797**
**B1**

(12) **EUROPÄISCHE PATENTSCHRIFT**

(45) Veröffentlichungstag der Patentschrift: **09.06.82**

(21) Anmeldenummer: **79100431.0**

(22) Anmeldetag: **13.02.79**

(51) Int. Cl.³: **G 11 B 27/32, A 61 B 5/04**

(54) Vorrichtung zur Verarbeitung von auf Magnetband gespeicherter Signalinformation.

(30) Priorität: **20.02.78 DE 2807097**
**20.02.78 DE 2807094**

(43) Veröffentlichungstag der Anmeldung:
**05.09.79 Patentblatt 79/18**

(45) Bekanntmachung des Hinweises auf die Patenterteilung:
**09.06.82 Patentblatt 82/23**

(84) Benannte Vertragsstaaten:
**BE CH DE FR GB IT NL SE**

(56) Entgegenhaltungen:
**DE - A - 2 633 371**
**DE - B - 2 213 075**

(73) Patentinhaber: **SIEMENS AKTIENGESELLSCHAFT**
**Berlin und München**
**Postfach 22 02 61**
**D-8000 München 22 (DE)**

(72) Erfinder: **Weigert, Kurt**
**Ernst-Heinkel-Weg 19**
**D-8500 Nürnberg (DE)**
Erfinder: **Kusserow, Bernd**
**Kulmbacher Strasse 12**
**D-8520 Erlangen (DE)**

Courier Press, Leamington Spa, England.

## Vorrichtung zur Verarbeitung von auf Magnetband gespeicherter Signalinformation

Die Erfindung bezieht sich auf eine Vorrichtung zur Verarbeitung von auf Magnetband gespeicherter Signalinformation zwecks Zeitvermessung von im wesentlichen periodischen Signalkomplexen, z.B. EKG, bestehend aus Aufnahmegerät zum Aufsprechen der Signalinformation auf Magnetband sowie Wiedergabegerät zum Auslesen der Signalinformation vom Magnetband mit Auswerteschaltung zur Analyse der Signalinformation, wobei das Aufnahmegerät einen Normtakt-Impulsegeber zum gleichzeitigen Aufsprechen von Normtakt-Impulsen und wobei das Wiedergabegerät eine separate Einheit zur Abnahme und Aufbereitung der auf dem Magnetband aufgesprochenen Normtakt-Impulse aufweist, so daß der Auswerteschaltung zur Signalanalyse die Normtakt-Impulse als mitlaufendes Zeitnormal zur Verfügung stehen.

In der modernen Medizintechnik wird zur Langzeitüberwachung die von einem Patienten abgenommene Signalinformation, wie beispielsweise das EKG, mittels eines Aufnahmegerätes auf Magnetband gespeichert. Dem Arzt ist dann jederzeit die Möglichkeit gegeben, mittels eines Wiedergabegerätes die gespeicherte Information abzurufen und bezüglich signifikanter Ereignisse auszuwerten. Dafür sind auch spezielle elektronische Vorrichtungen bekannt, die das im wesentlichen periodisch anfallende Signal hinsichtlich Amplitude und Breite charakteristischer Signalteile sowie Signalperiode (Frequenz) analysieren. Zur Aufnahme finden neuerdings am Körper des Patienten tragbare Aufnahmegeräte Verwendung, die mit Tonband-Normkassetten arbeiten. Derartige, am Körper des Patienten tragbare Aufnahmegeräte haben den Vorteil, daß die Signalabnahme vom Patienten kontinuierlich sowie ortsunabhängig erfolgen und daß der Patient sogar seinem normalen Tagesablauf nachgehen kann. Um eine möglichst lange Aufnahmezeit mit einer Tonbandkassette zu erreichen, ist man nun inzwischen dazu übergegangen, mit sehr geringen Bandaufnahmegeschwindigkeiten zu arbeiten. Die Wiedergabe und Auswertung ist dann wahlweise mit zeitgeraffter oder aufnahmesynchroner Wiedergabegeschwindigkeit möglich.

Besonders für die Möglichkeit der Aufnahme mit sehr geringen Aufnahmegeschwindigkeiten ($\leq 3,5$ mm/sec) und einer elektronischen Auswertung mit gegenüber der Aufnahmegeschwindigkeit sehr viel höheren Wiedergabegeschwindigkeiten wird die Forderung erhoben, die gespeicherte Signalinformation kohärent und ohne Verzerrung wiederzugeben. Aus der Unterhaltungselektronik (z.B. Photo-Technik und -Wirtschaft 24 (1973), 3, Seiten 62 bis 65) sind zwar Kopplungsverfahren für verschiedene Magnetträger bekannt, bei denen im Aufnahmeverfahren gleichzeitig mittels Taktgenerator auf die verschiedenen Informationsträger Taktsignale aufgezeichnet werden, aus denen im Wiedergabeverfahren mittels Koinzidenzvergleich Steuersignale für synchrone Wiedergabegeschwindigkeit abgeleitet werden. Aus der DE—AS 19 49 167 ist weiterhin eine Einrichtung zur Zeitbasisfehlerkorrektur bei Magnetbandaufnahmegeräten bekannt, bei dem durch Phasenvergleich eines zum Informationssignal zusätzlich aufgesprochenen Normtaktsignals mit dem Frequenzstandard eine Verzögerung für die das Informationssignal tragende Leitung gesteuert wird. Beim Stand der Technik haben die Normtaktsignale also im wesentlichen Steuer- bzw. Regelfunktionen für die Bandlaufgeschwindigkeit.

Speziell bei der Analyse von auf Magnetband gespeicherter EKG-Ableitungen als im wesentlichen periodischer Signalinformation machen sich aber schon geringste Zeitfehler bei Aufnahme und/oder Weidergabe der physiologischen Signale unmittelbar bei der Zeitvermessung bemerkbar und können so bei der weiteren Auswertung, beispielsweise im Sinne einer Impulsbreitenvermessung oder -abstandsmessung, im Ergebnis zu Fehldiagnosen führen. Derartige Zeitfehler lassen sich allerdings bei den bekannten Magnetbangeräten nich vollständig vermeiden. Sie werden durch die unvermeidbaren Gleichlaufschwankungen ("wow") des Magnetbandes bei der Aufnahme, die beispielsweise durch Erschütterungen, Temperaturschwankungen, Änderung der Betriebsspannung und weiteren Unzulänglichkeiten der Mechanik hervorgerufen werden, verursacht. Auch bei der bekannten Steuerung des Wiedergabegerätes mittels im Aufnahmeverfahren aufgesprochener Markierungsimpulse werden diese momentanen Gleichlaufschwankungen nicht eliminiert, sondern nur ausgeregelt. Zudem müssen speziell bei der zeitgerafften Wiedergabe und Auswertung mit gegenüber der Aufnahmegeschwindigkeit sehr viel höheren Geschwindigkeiten verschiedene elektronische Einheiten auf entsprechend kürzere Zeitkonstanten umgeschaltet und abgeglichen werden, wodurch erfahrungsgemäß weitere Fehler auftreten.

Der Erfindung liegt daher die Aufgabe zugrunde, eine Vorrichtung der eingangs genannten Art zu schaffen, bei der also die bei Aufnahme und/oder Wiedergabe aufgrund von Gleichlaufschwankungen des Magnetbandes nicht völlig vermeidbaren Zeitfehler eliminiert werden und bei der die auf dem Magnetband gespeicherte Signalinformation exakt ausgewertet wird, wobei ein schaltungsmäßig besonders einfacher Aufbau sichergestellt sein soll.

Die Aufgabe wird erfindungsgemäß dadurch gelöst, daß die Auswerteschaltung zur Signalanalyse speziell im Sinne einer Breitenvermessung und/oder Abstandsmessung der im wesentlichen periodischen Signalkomplexe durch Vergleich mit dem durch die mitlaufenden Normtakt-Impulse definierten Zeitnormal und damit unabhängig von sich bei Aufnahme und/oder Wiedergabe ändernden Laufgeschwindigkeiten des Magnetbandes wenigstens einen Zähler oder wenigstens einen Speicher zum Zählen und/oder Speichern anfallender Normtakt-Impulse aufweist, der über eine angeschlossene Steuerschaltung durch einen mit dem Auftreten eines signifikanten Signalereignisses ausgelösten Triggerimpuls einschaltbar ist und entweder durch einen am Ende des signifikanten Signalereignisses ausgelösten Triggerimpuls oder durch einen mit dem Auftreten eines zweiten signifikanten Signalereignisses ausgelöstem Triggerimpuls auf Null rücksetzbar ist. Dabei kann die Auswerteschaltung digital in der weise ausgelegt sein, daß der Zähler über ein Erkennungsglied für den Zählerstand eine bistabile Kippstufe in der Signalleitung ansteuert, die jeweils mit Auftreten eines signifikanten Signalereignisses von einem ersten in einen zweiten Zustand gesetzt und von einem Befehlimpuls des Erkennungsgliedes in den ersten Zustand zurückgesetzt wird, und daß das Kippzeitintervall der Schaltstufe die Zeitspanne zur Durchführung der Signalanalyse festlegt, wobei der Zähler durch Rücksetzen der Schaltstufe in den ersten Zustand getriggert auf Null zurücksetzbar ist. Die Auswerteschaltung kann aber auch analog in der Weise ausgelegt sein, daß als Speicher zwei aufladbare und über eine von aufeinanderfolgenden signifikanten Signalereignissen gesteuerte Schaltstufe verbindbare Speicher vorgesehen sind, von denen der erste mit Auftreten eines signifikanten Signalereignisses mittels der Normtaktimpulse aufladbar und unter gleichzeitiger Übernahme der Speicherspannung auf den zweiten Speicher mit demselben verbunden ist, und daß diese Umspeicherung durch das Auftreten eines weiteren signifikanten Signalereignisses über die Schaltstufe unterbrochen und der erste Speicher auf Null entladen wird.

Bei der erfindungsgemäßen Vorrichtung werden also in der Auswerteschaltung die bisher unvermeidbaren Fehler durch Gleichlaufschwankungen des Magnetbandes dadurch völlig beseitigt, daß die Normtaktimpulse, die im Aufnahmeverfahren derselben Gleichlaufschwankung wie die Signalinformation unterliegen, in der Auswerteschaltung als mitlaufendes Zeitnorm-Referenzsignal zur Verfügung stehen. Durch die direkte Steuerung der Auswerteschaltung mittels der Normtaktimpulse wird weiterhin bei der zeitgerafften Wiedergabe die Umschaltung der Zeitkonstanten verschiedener elektronischer Bauteile überflüssig, da die Frequenz- und Breitenvermessung direkt durch Vergleich mit den Normtaktsignalen erfolgen. Mit derartigen erfindungsgemäßen Vorrichtungen ist eine Auswertung alternativ zeitecht oder mit verschiedenen zeitgerafften Wiedergabegeschwindigkeiten (z.B. 30fach oder 60fach) möglich. Zusätzliche Abgleichfehler bei der Umschaltung sind dadurch also von vornherein ausgeschlossen. Normtaktimpulse und Signalinformation können wahlweise auf der gleichen Magnetspur oder bei Mehrspurbändern auf verschiedenen Spuren aufgenommen werden.

Die Aufzeichnungsart der Signalinformation (beispielsweise direkte (DR), frequenzmodulierte (FM) oder pulscodemodulierte Aufzeichnung (PCM) ist bei der erfindungsgemäßen Vorrichtung nicht von Bedeutung. Vorzugsweise werden aber die Signalinformation frequenzmoduliert und die Normtaktimpulse direkt aufgezeichnet. In der Auswerteschaltung können dann in vorteilhafter Weise die durch die Gleichlaufschwankungen der Bandlaufwerke modulierten Normtaktimpulse auf einen zusätzlichen FM-Demodulator gegeben und das so demodulierte Signal als Nachsteuersignal gegenphasig in einem FM-Demodulator für die Signalinformation eingespeist werden. Dadurch ist zusätzlich eine Amplitudenfehler- ("flutter")-Kompensation der Signalinformation bewirkt.

Mit der Auswerteschaltung zur Analyse von Signalinformationen sollen insbesondere signifikante Signalereignisse, beispielsweise QRS-Komplexe beim EKG, erfaßt und nach Lage und Breite vermessen werden. Daneben soll auch die Erfassung beispielsweise speziell im EKG auftretender Extrasystolen sowie Breitenvermessung und Klassifikation möglich sein. Im allgemeinen wird nun aber zur Erfassung der Extrasystolen nicht der gesamte RR-Abstand überwacht, sondern lediglich vom Arzt vorbestimmte Zeitintervalle innerhalb des RR-Abstandes. Signifikante Zeitintervalle sind dabei die Zeit bis zum Ende der T-Welle (RT-Zeit) sowie eine sogenannte "überwachte Periodenzeit" (ÜPZ kleiner als der RR-Abstand). Innerhalb solcher vorgebbarer Zeitintervalle des EKG's sind die vom Normverlauf abweichenden Signale (Extrasystolen) medizinisch klassifiziert. Je nach Lage und Breite unterscheidet man zwischen:

   a) Extrasystolen in der RT-Zeit,
   b) Extrasystolen in der ÜPZ,
   c) ventrikuläre Extrasystolen in der ÜPZ,
   d) supraventrikuläre Extrasystolen in der ÜPZ,
   e) ventrikuläre Extrasystolen,
   f) ventrikuläre Extrasystolen in Serie.

Mit der Erfindung ist es nun insbesondere möglich, in gewünschter Weise sämtliche, für die Durchführung der Signalanalyse notwendigen Zeitintervalle aus den Normtakt-Impulsen unabhängig von der Bandgeschwindigkeit bei Aufnahme und/oder Wiedergabe abzuleiten. Eine solche Auswertung erfolgt in besonders einfacher Weise digital durch Zählen der während des Signalereignisses anfallenden Normtakt-Impulse durch geeignete Zählglieder. Bei Verwendung vorprogrammierbarer Zählglieder können dementsprechend Überwachungsintervalle zeitgenau vorgegeben werden. Andererseits können auch analog arbeitende Frequenzmesser, bei denen beispielsweise Speicher vom periodischen Meßsignal gesteuert aufgeladen und über eine Signalperiode aufsummierte Spannungssignale festgehalten werden, gemäß der Erfindung betrieben werden. Dabei werden von den Normtakt-Impulsen Rechteckimpulse konstanter Ladungsmengen abgeleitet, mit denen der Speicher im Gegensatz zum Stand der Technik diskontinuierlich aufgeladen wird. Für eine Perioden- und/oder Frequenzmessung ist also durch Einspeisung der als Zeitnorm-Referenzsignale mitlaufenden Normtakt-Impulse ebenfalls eine Eliminierung von Zeitlauffehlern gewährleistet.

Weitere Vorteile und Einzelheiten der Erfindung ergeben sich anhand nachfolgender Figurenbeschreibung von in der Zeichnung dargestellten Ausführungsbeispielen. Es zeigen:

Fig. 1 die Prinzipdarstellung von Signalaufnahme und Wiedergabe mit Bandgeräten,

Fig. 2 ein Blockschaltbild zur Erzeugung eines definierten Zeitintervalles mittels Digitalschaltung,

Fig. 3 ein entsprechendes Blockschaltbild zur Breitenerfassung von signifikanten Signalkomplexen und

Fig. 4 ein Analogschaltbild zur Erzeugung einer aus einem EKG abgeleiteten perioden- bzw. frequenzproportionalen Spannung.

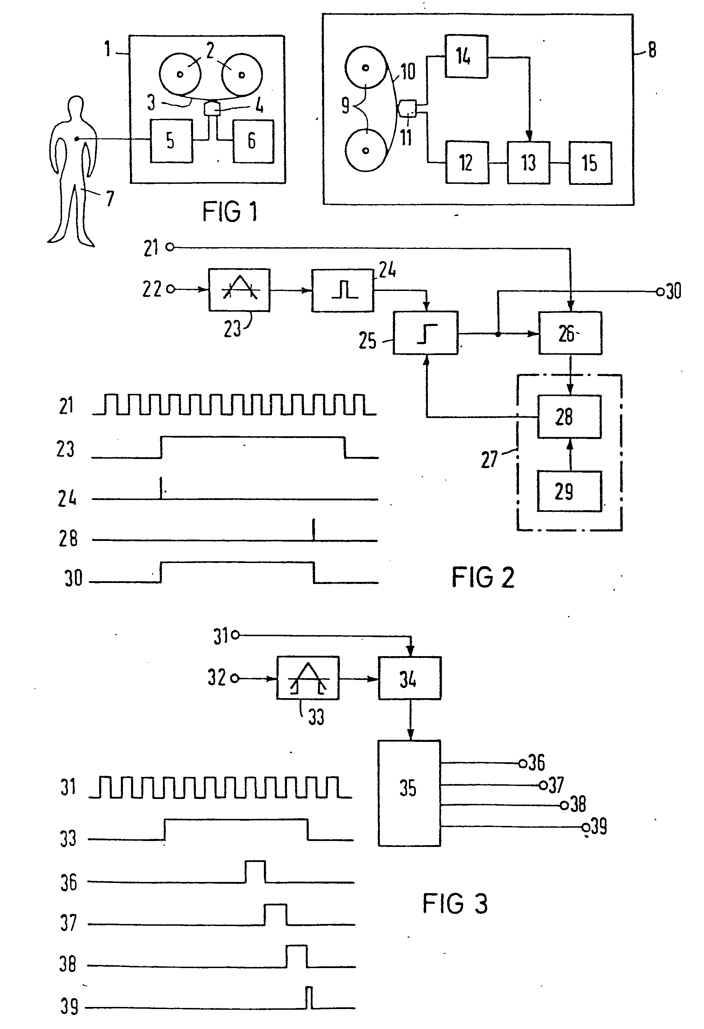

Zu den Fig. 2 bis 4 sind jeweils in zugehörigen Impulsdiagrammen die an den Schaltungspunkten auftretenden Spannungen dargestellt.

In der Fig. 1 ist mit 1 ein Aufnahmegerät bezeichnet, das ein Bandlaufwerk 2 mit Magnetband 3, einen Magnetkopf 4, einen Aufnahmeverstärker 5 und einen Taktimpulsgenerator 6 für Normtakt (z.B. 512 Hz) umfaßt. Der Magnetkopf 4 ist als mehrspuriger Aufnahmekopf für das zweispurige Magnetband 3 ausgebildet. Eine von einem Patienten 7 abgenommene Signalinformation wird über den Aufnahmeverstärker 5 als Signal dem Aufnahmekopf 4 und von diesem der ersten Spur des Magnetbandes 3 zugeführt. Gleichzeitig wird auf die zweite Spur das Normtaktsignal des Taktimpulsgenerators 6 aufgesprochen. Das zugehörige Wiedergabegerät ist mit 8 bezeichnet. Dieses umfaßt wiederum ein Laufwerk 9 für ein Magnetband 10 sowie einen entsprechend dem Aufnahmekopf 4 ausgebildeten mehrspurigen Wiedergabekopf 11. Die Signalinformation wird durch den Wiedergabekopf 11 vom Magnetband 10 ausgelesen, einer elektronischen Einheit zur Aufbereitung, Wiedergabe bzw. Darstellung 12 (beispielsweise einem Oszilloskop) zugeführt und anschließend einer Einheit 13 zur Auswertung der Signalinformation eingespeist. Gleichzeitig werden vom Wiedergabekopf 11 die Normtaktimpulse ausgelesen und nach Aufbereitung in der Wiedergabeeinheit 14 ebenfalls der Auswerteeinheit 13 eingespeist. In jeder Auswerteperiode steht dann der Auswerteeinheit 13 ein entsprechendes Zeit-Bezugssignal zur Verfügung, das relativ entsprechend den Gleichlaufschwankungen im Aufnahmegerät 1 verändert ist, aber jeweils ein Zeitnormal für die elektronische Auswerteschaltung definiert. Nach Auswertung des Signals bezüglich der signifikanten Größen werden diese einer Anzeigeneinheit 15 angezeigt.

In der Fig. 2 sind mit 21 und 22 Signaleingänge bezeichnet. Über den Signaleingang 21 werden die von einer ersten Spur des Magnetbandes abgenommenen Normtaktimpulse, die das Zeitnormal für die Auswerteschaltung definieren sollen, eingespeist; auf den Eingang 22 wird das EKG gegeben. Mittels R-Zackendiskriminator 23 wird bei Auftreten eines QRS-Komplexes ein Rechtecksignal definierter Höhe und Breite erzeugt. QRS-Komplexe oder Extrasystolen werden erkannt, wenn ein bestimmter, vorgebbarer Schwellwert (Amplitude) und eine bestimmte Basisbreite des Signals, beispielsweise 12 ms, überschritten wird. Dieser Wert dient zur Unterdrückung von Pacemaker-, Markierungs- oder sonstigen Störimpulse. Das Rechtecksignal triggert einen monostabilen Multivibrator 24 und eine nachfolgende bistabile Schaltstufe 25 (Flip-Flop). Mit der Vorderflanke des Rechteckimpulses wird vom Multivibrator 24 ein Markierungsimpuls erzeugt und gleichzeitig das Flip-Flop 25 von einem ersten in einen zweiten Zustand gesetzt. Bei gesetztem Flip-Flop 25 wird ein Zähler 26, der von der Eingangsleitung 21 zum Zählen anfallender Normtaktimpulse angesteuert wird, eingeschaltet. Bei nicht gesetztem Flip-Flop 25 ist der Zähler 26 automatisch auf Null zurückgesetzt, so daß bei Aufheben der Reset-Funktion der Zählvorgang immer bei Null gestartet wird. Der Zählerstand des Zählers 26 wird auf eine logische Erkennungseinheit 27 mit einem Komparator 28 gegeben, der von einem Programmschalter 29 angesteuert wird. Komparator 28 und Programmschalter 29 bilden also zusammen die logische Erkennungseinheit 27. Mit dem Programmschalter 29 kann vom Benutzer ein Zeitintervall extern angewählt werden, das dem Komparator 28 als binäres Vergleichszählsignal zugeleitet wird. Der Komparator 28 vergleicht den Zählerstand mit der angewählten Information und gibt bei Übereinstimmung ein Befehlssignal auf das Flip-Flop 25 zurück, das dann in den ersten Zustand zurückgesetzt wird. Gleichzeitig mit dem Flip-Flop 25 wird der Zähler 26 zurückgesetzt. Das angewählte Kippzeitintervall des Flip-Flops 25 wird am Ausgang 30 zur Weiterverarbeitung ausgegeben.

Im zugehörigen Impulsdiagramm geben die Bezugszeichen die Schaltpunkte im Blockschaltbild an. Da am Programmschalter 29 der Logikeinheit 27 effektiv ein Zählerstand eingegeben wird, spielt die Wiedergabegeschwindigkeit des Magnetbandes keine Rolle. Es wird lediglich die entsprechende Anzahl von Normtaktimpulsen aufsummiert.

Am Eingang 22 wird vom R-Zacken-Diskriminator 23 bereits ein Impuls definierter Länge abgegeben, für den sich eine Breite von 140 ms als geeignet erwiesen hat. Dieser Impuls wird zweckmäßigerweise ebenfalls durch aufsummierte Normtaktimpulse zeitgenau abgeleitet. Dazu ist im Prinzip eine weitere Schaltung entsprechend Fig. 2 notwendig, bei der jedoch ein separater Programmschalter entfällt. Dafür ist der Komparator 28 als Logikeinheit fest vorprogrammiert.

In der Fig. 3 sind mit 31 und 32 Signaleingänge bezeichnet. Über Eingang 31 werden — entsprechend Fig. 2 — die Normtaktimpulse, über Eingang 32 ein vom EKG mittels eines Schwellwertdiskriminators 33 abgeleitetes Rechtecksignal auf einen Zähler 34 — entsprechend der Fig. 2 — gegeben. Der Schwellwertdiskriminator 33 erzeugt ein Rechtecksignal, dessen Breite der Basisbreite des zu vermessenden Komplexes proportional ist. Die Vorderflanke dieses Eingangssignales hebt wiederum die Reset-Funktion des Zählers 34 auf, so daß nun die Normtaktimpulse aufsummiert werden. Zähler 34 steuert eine Logikeinheit 35 an. In dieser Logikeinheit 35 sind bestimmte diskrete Ausgänge in Abhängigkeit vom Zählerstand des Zählers 34 fest verschaltet. Es sind die Ausgänge 36 bis 39 angegeben, die zu den Zeiten $t_1$ bis $t_4$ aktiviert werden. Aus dem Impulsdiagramm zu Fig. 3 ist die Schaltung der Logikeinheit 25 ersichtlich:

Die Ausgänge 36 bis 39 werden hier speziell in den Zeitintervallen, in denen Signalbreiten erwartet werden, fortlaufend aktiviert. Natürlich können in anderen Ausführungsbeispielen die Ausgänge entsprechend den speziellen Bedürfnissen verschaltet sein.

Vom Benutzer wird nun einer der Ausgänge — beispielsweise über einen separaten Schalter — angewählt. Dadurch wird also ein Zeitintervall vorgegeben. Wird das vorgegebene Zeitintervall vom Eingangssignal durch den aufsummierten Zählerstand erreicht, so ist der vorher angewählte Ausgang aktiviert. Das Eingangssignal ist dann also in seiner Breite klassifiziert und kann anschließend weiterverarbeitet werden. Beispielsweise werden Signale auf einem separaten Zähler gezählt. Dem Benutzer steht dann als Information die Ereignishäufigkeit von klassifizierten Signalkomplexen zur Verfügung.

Entscheidend ist, daß die Klassifikation der Eingangssignale allein durch Vergleich mit den Normtaktsignalen und damit von der Wiedergabegeschwindigkeit unabhängig erfolgt. Die bei Umschaltung der Bandgeschwindigkeiten durch die verschiedenen Zeitkonstanten verursachten Fehler werden eliminiert. Die Genauigkeit der mit der erfindungsgemäßen Vorrichtung durchgeführten Auswertung ist lediglich abhängig vom Verhältnis der zu bildenden Zeitintervalle bzw. der zu messenden Zeiten zur Frequenz der Normtaktimpulse. Da diese Frequenz gegenüber der Frequenz der beim EKG auszuwertenden Signalfolgen sehr groß ist, ist die erfindungsgemäße Auswertung wesentlich genauer als mit den bisher bekannten Vorrichtungen.

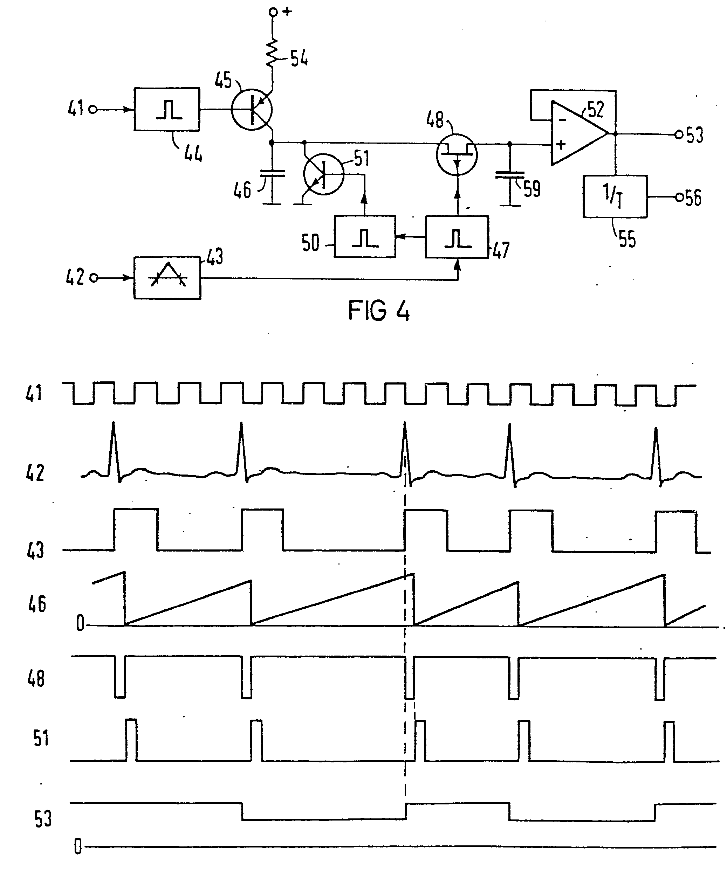

In der Fig. 4 sind mit 41 und 42 Signaleingänge bezeichnet. Über den Signaleingang 41 werden der Zeitmeßschaltung die von einer separaten Spur des Magnetbandes ausgelesenen Normtaktimpulse, die das Zeitnormal für die Auswerteschaltung definieren sollen, eingespeist; der Eingang 42 nimmt das mit dem EKG korrespondierende Eingangssignal auf. Durch einen R-Zackendiskriminator 43 wird bei Auftreten eines QRS-Komplexes ein Rechteckimpuls erzeugt. Die Normtaktimpulse steuern über Eingang 41 einen ersten monostabilen Multivibrator 44 an, der mit Rechteckimpulsen gleicher Breite (also mit konstanter Ladungsmenge pro Impuls) über einen pnp-Transistor 45 in Emitterschaltung stromkonstant einen mit Massepotential verbundenen Kondensator 46 auflädt. Die Emitterseite des Transistors 45 ist über einen Widerstand 54 auf positives Potential angeschaltet.

Dabei ist der Multivibrator 44 so gewählt, daß die erzeugten Ladeimpulse kürzer sind als die bei höchstmöglicher Bandgeschwindigkeit kürzeste Folgedauer der Normtaktimpulse. Geht man beispielsweise von einem Normtakt mit 500 Hz aus, so muß für 60fache Wiedergabegeschwindigkeit der Ladeimpuls kürzer als ca. 30 $\mu$s sein. Im Gegensatz zur üblichen stromkonstanten Aufladung wird der Kondensator 46 also "quantisiert" durch den Normtakt getriggert aufgeladen. Die geglättete Anstiegssteilheit der Speicherspannung des Kondensators 46 hängt nun von der Anzahl der Rechteckimpulse des Monoflops 44 pro Zeiteinheit — also nur von der definierten Normtaktimpulsfolge — ab. Vom Triggersignal des R-Zacken-Diskriminators 43 wird über einen zweiten monostabilen Multivibrator 47 ein Feldeffekttransistor 48 angesteuert, der die Funktion eines Schalters hat. Bei geöffnetem Feldeffekttransistor 48 wird die augenblickliche Speicherspannung vom Kondensator 46 auf einen zweiten Kondensator 59 umgeladen. Die Kapazität von Kondensator 46 ist dabei groß gegen die Kapazität von Kondensator 59 ($C_2 \ll C_1$), so daß eine wirksame Umladung lediglich in eine Richtung erfolgen kann. Gegebenenfalls ist noch ein Trennverstärker zwischen die Kondensatoren geschaltet. Nach erneuter Sperrung des Feldeffekttransistors 48 wird über einen dritten monostabilen Multivibrator 50, der hinter den zweiten monostabilen Multivibrator 47 geschaltet ist und von diesem getriggert wird, ein npn-Transistor 51 in Emitterschaltung, dessen Emitterseite mit Massepotential verbunden ist, durchgesteuert. Über den leitend gemachten Transistor 51 kann dann der erste Kondensator 46 auf Null entladen werden, um mit anschließender Sperrung des Transistors 51 wieder von den diskreten Normtaktimpulsen getriggert durch Impulse konstanter Ladungsmenge neu aufgeladen zu werden.

Es wird also am Kondensator 46 eine analog zur Signalfolge von der R-Zacke getriggerte (diskontinuierlich ansteigende) Sägezahnspannung erzeugt, deren Amplitude dem RR-Abstand proportional ist. Die Endspannung ist allein von der Anzahl der im Periodenintervall auftretenden Normtaktimpulse abhängig und daher von der Bandgeschwindigkeit beim Auslesen des Magnetbandes unabhängig. Die Ladespannung wird nach Umladung auf Kondensator 49 von diesem auf einen hochohmigen Verstärker 52 gegeben, der am Ausgang 53 eine dem Signalabstand proportionale Spannung liefert. Über ein Rechengleid 55 wird gegebenenfalls am Ausgang 56 daraus ein Frequenzmeßwert angezeigt.

Mit der in Fig. 4 beschriebenen Schaltung ist also eine bandgeschwindigkeitsunabhängige Perioden- bzw. Frequenzmessung von im wesentlichen periodischen Impulsfolgen der auf Magnetband gespeicherten Signalinformation in einfacher Weise möglich. Dabei erfolgt die Auswertung zunächst als "beat-to-beat"-Messung, wobei bei der EKG-Auswertung in vorteilhafter Weise auch einzeln auftretende Arrhythmien erfaßt werden können.

Die erfindungsgemäßen Vorrichtungen wurden speziell für die Auswertung von EKG-Signalfolgen beschrieben. Natürlich sind mit diesen Vorrichtungen auch andere periodische Signalfolgen, beispielsweise EEG, Blutdruck oder Atemsignale, die auf Magnetband aufgenommen wurden, zeitgenau auswertbar.

**Patentansprüche**

1. Vorrichtung zur Verarbeitung von auf Magnetband gespeicherter Signalinformation zwecks Zeitvermessung von im wesentlichen periodischen Signalkomplexen, z.B. EKG, bestehend aus Aufnahmegerät (1) zum Aufsprechen der Signalinformation auf Magnetband (3) sowie Wiedergabegerät (8) zum Auslesen der Signalinformation vom Magnetband mit Auswerteschaltung zur Analyse der Signalinformation, wobei das Aufnahmegerät einen Normtakt-Impulsgeber (6) zum gleichzeitigen Aufsprechen von Normtaktimpulsen und wobei das Wiedergabegerät eine separate Einheit (14, 13) zur Abnahme und Aufbereitung der auf dem Magnetband aufgesprochenen Normtakt-Impulse aufweist, so daß der Auswerteschaltung zur Signalanalyse die Normtakt-Impulse als mitlaufendes Zeitnormal zur Verfügung stehen, dadurch gekennzeichnet, daß die Auswerteschaltung (13) zur Signalanalyse speziell im Sinne einer Breitenvermessung und/oder Abstandsmessung der im wesentlichen periodischen Signalkomplexe durch Vergleich mit dem durch die mitlaufenden Normtakt-Impulse definierten Zeitnormal und damit unabhängig von sich bei Aufnahme und/oder Wiedergabe ändernden Laufgeschwindigkeiten des Magnetbandes (3, 10) wenigstens einen Zähler (26, 34) oder wenigstens einen Speicher (45—59) zum Zählen und/oder Speichern anfallender Normtakt-Impulse aufweist, der über eine angeschlossene Steuerschaltung (23—25; 33) durch einen mit dem Auftreten eines signifikanten Signalereignisses ausgelösten Triggerimpuls einschaltbar ist und entweder durch einen am Ende des signifikanten Signalereignisses ausgelösten Triggerimpuls (Fig. 3) oder durch einen mit dem Auftreten eines zweiten signifikanten Signalereignisses ausgelösten Triggerimpuls (Fig. 4) auf Null rücksetzbar ist.

2. Vorrichtung nach Anspruch 1, dadurch gekennzeichnet, daß der Zähler (26) über ein Erkennungsglied (27) für den Zählerstand eine bistabile Schaltstufe (25) in der Signalleitung ansteuert, die jeweils mit Auftreten eines signifikanten Signalereignisses von einem ersten in einen zweiten Zustand gesetzt und von einem Befehlsimpuls des Erkennungsgliedes (27) in den ersten Zustand zurückgesetzt wird, und daß das Kippzeitintervall der Schaltstufe (25) die Zeitspanne zur Durchführung der Signalanalyse festlegt, wobei der Zähler (26, 34) durch das Rücksetzen der Schaltstufe (25) in den ersten Zustand getriggert auf Null zurücksetzbar ist (Fig. 2).

3. Vorrichtung nach Anspruch 2, dadurch gekennzeichnet, daß die bistabile Schaltstufe (25) von einem Diskriminator (23) für das signifikante Signalereignis über einen monostabilen Multivibrator (24) angesteuert wird.

4. Vorrichtung nach Anspruch 1, dadurch gekennzeichnet, daß der Zähler (34) eine logische Schaltung (35) mit mehreren diskreten Ausgängen (36 bis 39) ansteuert, die abhängig vom Zählerstand aktiviert werden (Fig. 3).

5. Vorrichtung nach Anspruch 4, dadurch gekennzeichnet, daß ein den Zähler (34) ansteuernder Schwellwertdiskriminator (33) ein der Breite des signifikanten Signalereignisses entsprechendes Normsignal erzeugt, das die Zeitspanne zum Zählen der Normtaktsignale festlegt.

6. Vorrichtung nach Anspruch 1, dadurch gekennzeichnet, daß als Speicher zwei aufladbare und über eine von aufeinanderfolgenden signifikanten Signalereignissen gesteuerte Schaltstufe (47, 48) verbindbare Speicher (46, 59) vorgesehen sind, von denen der erste (46) mit Auftreten eines signifikanten Signalereignisses mittels der Normtaktimpulse aufladbar und unter gleichzeitiger Übernahme der Speicherspannung auf den zweiten Speicher (59) mit demselben verbunden ist, und daß diese Umspeicherung durch das Auftreten eines weiteren signifikanten Signalereignisses über die Schaltstufe (47, 48) unterbrochen und der erste Speicher (46) auf Null entladen wird (Fig. 4).

7. Vorrichtung nach Anspruch 6, dadurch gekennzeichnet, daß die diskreten Ladeimpulse zur Aufladung des ersten Speichers (46) von einem von den Normtaktimpulsen angesteuerten ersten monostabilen Multivibrator (44) erzeugt werden, wobei die Rechteckimpulse kürzer sind als die bei höchstmöglicher Bandgeschwindigkeit auftretende kürzeste Folgezeit der Normtaktimpulse.

8. Vorrichtung nach Anspruch 1 und 6, dadurch gekennzeichnet, daß die aufladbaren Speicher Kondensatoren (46, 59) sind, von denen der erste Kondensator (46) über eine dazu parallel liegende und über die Schaltstufe (47, 48) gesteuerte zweite Schaltstufe (51) separat entladbar ist, wobei die Kapazität des zweiten Kondensators (49) klein gegen die des ersten Kondensators (46) ist ($C_2 \ll C_1$), so daß die Umladung des zweiten (59) auf den ersten Kondensator (46) keine merkliche Ladungsänderung des ersten Kondensators (46) bewirkt.

9. Vorrichtung nach Anspruch 8, dadurch gekennzeichnet, daß die Schaltstufe zwischen den Kondensatoren (46, 59) durch einen Feldeffekttransistor (48) gebildet ist, der von einem Diskriminator (43) für das signifikante Signalereignis über einen zweiten monostabilen Multivibrator (47) zur Spannungsübernahme durchgeschaltet wird.

10. Vorrichtung nach Anspruch 8 und 9, dadurch gekennzeichnet, daß der zweite monostabile Multivibrator (47) mit seiner Rückflanke einen dritten monostabilen Multivibrator (50) triggert, der einen an den ersten Kondensator (46) angeschalteten Transistor (51) als dazu parallel liegende Schaltstufe durchschaltet, so daß der erste Kondensator (46) separat entladen wird.

11. Vorrichtung nach Anspruch 8, dadurch gekennzeichnet, daß die vom zweiten Kondensator (59) gespeicherte Ladespannung über einen hochohmigen Verstärker (52) und gegebenenfalls ein Rechenglied (55) als perioden- bzw. frequenzproportionales Analogsignal anzeigbar ist.

## Claims

1. Device for the processing of signal information stored on magnetic tape for the purpose of time measurement of fundamentally periodical signal complexes, e.g. ECG, consisting of the recorder (1) for the recording of the signal information onto the magnetic tape (3), and of the reproduction device (8) for reading out the signal information from the magnetic tape with an analysis circuit for the analysis of the signal information, where the recorder comprises a standard clock pulse generator (6) for the simultaneous recording of standard clock pulses, and where the reproduction device comprises a separate unit (14, 13) for the withdrawal and preparation of the standard clock pulses which have been recorded on the magnetic tape, so that the standard clock pulses are available, as a concurrent time reference, to the analysis circuit for the purpose of signal analysis, characterised in that for signal analysis, in particular in the sense of a width measurement and/or spacing measurement of a fundamentally periodical signal complex by comparison with the time reference defined by the concurrent standard clock pulses and thus independently of changes in the running speeds of the magnetic tape (3, 10) which occur during recording and/or reproduction, the analysis circuit (13) comprises at least one counter (26, 34) or at least one store (45—59) for counting and/or storing occurring standard clock pulses, which can be switched on via a connected control circuit (23—25; 33) by means of a trigger pulse which is triggered on the occurrence of a significant signal event, and can be reset to zero either by means of a trigger pulse which is triggered at the end of the significant signal event (Fig. 3), or by means of a trigger pulse which is triggered on the occurrence of a second significant signal event (Fig. 4).

2. Device as claimed in claim 1 characterised in that via a recognition element (27) for the count, the counter (26) operates a bistable switching stage (25) in the signal line which, on the occurrence of a significant signal event, is brought from a first into a second state and is reset into the first state by means of a command pulse from the recognition element (27), and that the switched time interval of the switching stage (25) determines the time required to effect the signal analysis, where the counter (26, 34) can be reset to zero by resetting the switching stage (25) into the first stage (Fig. 2).

3. Devices as claimed in claim 2, characterised in that the bistable switching stage (25) is operated by a discriminator (23) for the significant signal event via a monostable multivibrator (24).

4. Device as claimed in claim 1, characterised in that the counter (34) operates a logic circuit (35) having a plurality of discrete outputs (36 to 39) which are activated in dependence upon the counter (Fig. 3).

5. Device as claimed in claim 4, characterised in that a threshold value discriminator (33) which operates the counter (34) produces a reference signal which corresponds to the width of the significant signal event and which determines the length of time for the counting of the standard clock signals.

6. Device as claimed in claim 1, characterised in that the store consists of two chargeable stores (46, 59) which can be connected via a switching stage (47, 48) controlled by consecutive, significant signal events. the first of which (46) can be charged by means of the standard clock pulses on the occurrence of a significant signal event and, simultaneously to the transfer of the store voltage to the second store (59), is connected thereto, and that this restorage process is interrupted via the switching stage (47, 48) as a result of the occurrence of a further significant signal event, and the first store (46) is discharged to zero (Fig. 4).

7. Device as claimed in claim 6. characterised in that the discrete charging pulses which serve to charge the first store (46) are produced by a first monostable multivibrator (44) which is operated by the standard clock pulses, where the rectangular pulses are shorter than the shortest sequence time of the standard clock pulses which occurs at the highest possible tape speed.

8. Device as claimed in claim 1 and 6, characterised in that the chargeable stores are capacitors (46, 59), the first of which (46) can be separately discharged via a parallel, second switching stage (51) which is controlled via the switching stage (47, 48), where the capacitance of the second capacitor (49) is small in relation to that of the first capacitor ($C_2 \ll C_1$), so that the recharging of the second capacitor (59) by the first capacitor (46) does not result in any noticeable change in the charge of the first capacitor (46).

9. Device as claimed in claim 8, characterised in that the switching stage between the capacitors (46, 59) is formed by a field effect transistor (48) which is switched through for purposes of voltage transfer by a discriminator (43) for the significant signal event via a second monostable multivibrator (47).

10. Device as claimed in claim 8 and 9, characterised in that with its rear flank the second monostable multivibrator (47) triggers a third monostable multivibrator (50) which switches through a transistor (51), connected to the first capacitor (46), as parallel switching stage, so that the first capacitor (46) is separately discharged.

11. Device as claimed in claim 8, characterised in that the charge voltage stored by the second capacitor (59) can be displayed as an analogue signal proportional to the period or frequency via a highly ohmic amplifier (52) and possibly a calculating element (55).

**Revendications**

1. Installation de traitement d'une information, sous forme de signaux, mémorisée sur bande magnétique en vue de mesurer dans le temps des complexes de signaux essentiellement périodiques, par exemple des électrocardiogrammes, se composant d'un appareil (1) enregistreur destiné à enregistrer l'information sous forme de signaux sur une bande (3) magnétique, ainsi que d'un appareil (8) de restitution, destiné à lire sur la bande l'information sous forme de signaux et ayant un montage d'évaluation destiné à analyser l'information sous forme de signaux, l'appareil enregistreur comprenant un émetteur (6) d'impulsions d'horloge destiné à enregistrer simultanément des impulsions à cadence normalisée, tandis que l'appareil (8) de restitution comprend une unité (14, 13) distincte destinée à recevoir et à traiter les impulsions d'horloge enregistrées sur la bande magnétique de manière à mettre à disposition du montage d'évaluation, en vue de l'analyse des signaux, les impulsions d'horloge en tant qu'étalon de temps d'accompagnement, caractérisée en ce que le montage (13) d'évaluation, destiné à analyser les signaux, spécialement en vue de mesurer la largeur et/ou l'écartement de complexes de signaux essentiellement périodiques par comparaison à l'étalon de temps défini par les impulsions à cadence normalisée d'accompagnement et donc indépendamment des vitesses de passage de la bande (3, 10) magnétique qui se modifient à l'enregistrement et/ou à la restitution, comporte au moins un compteur (26, 34) ou au moins une mémoire (45—59), qui est destiné à compter et/ou à mémoriser les impulsions à cadence normalisée qui se produisent, et qui peut être branché par l'intermédiaire d'un circuit (23—25; 33) de commande sous l'action d'une impulsion de déclenchement déclenchée par l'apparition d'un événement significatif en forme de signal et qui peut être remis à zéro soit par une impulsion de déclenchement (figure 3) déclenchée à la fin de l'événement significatif soit par l'apparition d'un second événement significatif sous forme de signal (figure 4).

2. Installation suivant la revendication 1, caractérisée en ce que le compteur (26) commande, par l'intermédiaire d'un organe (27) de reconnaissance de l'état du compteur, un étage (25) de commutation bistable sur le conducteur pour les signaux, lequel est mis, lors de l'apparition d'un événement significatif, sous forme de signaux d'un premier à un second état et est remis dans le premier état par une impulsion de commande de l'organe (27) de reconnaissance, et la durée de basculement de l'étage (25) fixe la durée de l'analyse des signaux, le compteur (26, 34) pouvant être remis à zéro par la remise dans le premier état de l'étage (25) (figure 2).

3. Installation suivant la revendication 2, caractérisée en ce que l'étage (25) bistable est commandé par un discriminateur (23) pour l'événement significatif sous forme de signaux par l'intermédiaire d'un multivibrateur monostable (24).

4. Installation suivant la revendication 1, caractérisée en ce que le compteur (34) commande un circuit (35) logique ayant plusieurs sorties (36 à 39) discrètes qui sont attaquées en fonction de l'état du compteur (figure 3).

5. Installation suivant la revendication 4, caractérisée en ce qu'un discriminateur (33) de valeur de seuil commandant le compteur (34) fournit un signal normalisé correspondant à la largeur de l'événement significatif sous forme de signal, et déterminant la durée pour compter les signaux à cadence normalisée.

6. Installation suivant la revendication 1, caractérisée en ce qu'il est prévu comme mémoire deux mémoires (46, 59) qui peuvent être chargées et qui peuvent être reliées par un étage (47, 48) de commutation commandé par des événements significatifs successifs sous forme de signaux, la première mémoire (46) pouvant être chargée au moyen des impulsions à cadence normalisée et pouvant être reliée à la seconde mémoire (59) avec transfert simultané de la tension de mémorisation à la seconde mémoire (59) et ce transfert de mémoire est interrompu par l'intermédiaire de l'étage (47, 48) de commutation, lors de l'apparition d'un autre événement significatif sous forme de signaux, la première mémoire (46) étant déchargée à zéro (figure 4).

7. Installation suivant la revendication 6, caractérisée en ce que les impulsions de charge discrètes destinées à charger la première mémoire (46) sont obtenues par un premier multivibrateur (44) monostable commandé par l'une des impulsions à cadence normalisée, les impulsions rectangulaires étant plus courtes que l'intervalle de temps le plus court qui sépare les impulsions à cadence normalisée lorsque la vitesse de la bande est la plus élevée possible.

8. Installation suivant la revendication 1 ou 6, caractérisée en ce que les mémoires qui peuvent être décharges sont des condensateurs (46, 59), le premier (46) d'entre eux pouvant être déchargé séparément par un second étage (51) de commutation en parallèle avec le condensateur et commandé par l'étage (47, 48) de commutation, la capacité du second condensateur (44) étant petite par rapport à celle du premier (46) $(C_2 \ll C_1)$ de manière à ce que la décharge du second condensateur (59) dans le premier (46) ne provoque pas de variation notable de la charge du premier condensateur (46).

9. Installation suivant la revendication 8, caractérisée en ce que l'étage de commutation entre les condensateurs (46, 59) est constitué par un transistor (48) à effet de champ qui est rendu passant par un discriminateur (43) de l'événément significatif sous forme de signaux par l'intermédiaire d'un deuxième multivibrateur (47) monostable, pour une prise en charge de la tension.

10. Installation suivant le revendication 8 et 9, caractérisée en ce que le deuxième multivibrateur (47) monostable déclenche, par son front arrière, un troisième multivibrateur (50) monostable qui rend passant un transistor (51) relié au premier condensateur (46) et servant d'étage de commutation en parallèle à celui-ci, de sorte que le premier condensateur (46) peut être déchargé séparément.

11. Installation suivant la revendication 8, caractérisée en ce que la tension de charge emmagasinée par le second condensateur (59) peut être affichée par l'intermédiaire d'un amplificateur (52) à forte résistance ohmique et, le cas échéant, par l'intermédiaire d'un organe (55) de calcul, sous la forme d'un signal analogique proportionnel à la période ou à la fréquence.

FIG 1

FIG 2

FIG 3

FIG 4